# EUROPEAN PATENT APPLICATION

(11) **EP 3 495 345 A1**
(43) Date of publication of application: **12.06.2019**
(21) Application number: 17205605.3
(22) Date of filing: 06.12.2017
(51) Int. Cl.: C07C 231/10, C08G 18/00, C08L 75/00

(54) **PROCESS FOR THE PREPARATION OF DI- OR POLYFORMAMIDES**

(71) Applicant: Covestro Deutschland AG, 51373 Leverkusen (DE)
(72) Inventor: The designation of the inventor has not yet been filed
(74) Representative: Levpat

(57) **Abstract**

The invention relates to a process for preparing at least one di- or polyformamide comprising at least two -NHCHO groups by reacting at least one primary di- or polyamine with carbon dioxide in the presence of hydrogen and at least one catalyst selected from the group consisting of heterogeneous and homogeneous catalysts or mixtures thereof. The invention further relates to a di- or polyformamide and their use for different purposes as well as a two-component-system comprising the di- or polyformamide.

## Description

The present invention relates to a process for the preparation of di- or polyformamides by reaction of the corresponding amines with carbon dioxide (CO₂) in the presence of hydrogen (H₂).

Polyurethane polymers are well known and used in a wide variety of applications from structural and foamed articles to coatings. These polymers are prepared by reactions of polyisocyanates with compositions containing a compound having at least two Zerewitinoff active hydrogen atoms. The physical properties of the polyurethane polymers are to a wide extent defined by these compositions. Often, amine chain extenders are used in order to introduce urea groups into the polymer which lead to so-called hard segments which have beneficial effects on the physical properties of the polymer.

Also pure polyurea polymers are known which are formed by the reaction of polyisocyanates and polyamines.

A major disadvantage of amine chain extenders is the high reactivity of the amines caused by their high nucleophilicity. There are a number of less reactive amines commercially available. However, most of them are aromatic amines due to their lower nucleophilicity compared to aliphatic amines. To achieve even less reactivity, most of them are substituted aromatic amines like 4,4'-methylenebis(2-chloroaniline), diethyltoluylene diamine or dimethylthio toluylene diamine. Unfortunately, such aromatic diamines cause discoloration of the polymers which is unwanted in many applications and they still react fast with isocyanates, especially with aromatic isocyanates.

Some aliphatic amines containing Zerewitinoff active hydrogen atoms are also known to have a lower reactivity, as for example polyaspartics which can be obtained by addition reactions of diamines and maleic acid. Such amines are particularly useful in coating applications, where discoloration is unwanted.

Another class of less reactive aliphatic amines containing at least two Zerewitinoff active hydrogen atoms is the class of formamide terminated compounds. In WO 2011/006607 A2 binder systems are described that contain such formamide containing compounds. In particular, various routes for the preparation of the formamide terminated compound are disclosed, which start with the corresponding di- and/or polyamines. The mentioned reactants comprise formic acid or formic-acid derivatives such as formic-acid esters, amides, anhydrides or carbon monoxide. An alternative disclosed route to formamide terminated compounds proceeds via alkylation of formamide in presence of strong bases that lead to the formation of formamide anions. However, the preferred route to formamide terminated compounds is the reaction of a diamine and excess formic-acid alkyl ester, while removing the formed alkyl alcohols.

EP 2 275 467 A1 describes NCO-terminated prepolymers derived from formamide terminated compounds and isocyanates. The disclosed route for the formation of the formamide terminated compounds proceeds via formic acid ethyl ester with removal of excess ester and the formed ethanol.

Interestingly, the reactants formic acid and formic-acid derivatives can be obtained from the renewable raw material carbon dioxide (CO₂). Indeed, the usage of CO₂ as a renewable C1-builing block is strongly desired in the chemical industry.

However, incorporating CO₂ as a raw material in the diformamide synthesis via the reactants formic acid or formic-acid derivatives requires additional synthesis and purification steps. Such steps complicate the overall process. Thus, it is desirable to find a route that directly uses CO₂ as a reactant in the synthesis of di- or polyformamides, which contain Zerewitinoff active hydrogen atoms.

Therefore, it was the object of the present invention to provide a direct process for the preparation of di- or polyformamides containing Zerewitinoff active hydrogen atoms that starts from primary di- or polyamines and that uses CO₂ as a reactant.

The present invention therefore provides a process for preparing at least one di- or polyformamide comprising at least two -NHCHO groups by reacting at least one primary di- or polyamine with carbon dioxide in the presence of hydrogen and at least one catalyst selected from the group consisting of one or more heterogeneous and homogeneous catalysts or mixtures thereof.

According to this application the term "-NHCHO" stands for a formamide group containing a Zerewitinoff active hydrogen atom attached to the nitrogen atom of the respective formamide group.

According to the invention, primary di- or polyamines are such amines that contain two or more groups described by the formula -NH₂ attached to an organic backbone. The backbone can be aromatic as in the isomers of toluylendiamine (TDA), the isomers of diaminodiphenylmethane (MDA), the higher homologues of MDA, 1,5-naphthalenediamine (NDA), or 1,3- and/or 1,4-diaminobenzene. It can also be araliphatic as for example in m-xylylenediamine or p-xylylenediamine and it can be aliphatic or cycloaliphatic as in ethylenediamine, 1,2-diaminopropane, 1,3-diaminopropane, 1,4-diaminobutane, neopentanediamine, 1,5-diaminopentane (PDA), 1,5-diamino-2-methylpentane, 2-butyl-2-ethyl-1,5-pentanediamine, 1,6-diaminohexane (HDA), 2,5-diamino-2,5-dimethylhexane, 2,2,4- and/or 2,4,4-trimethyl-1,6-diaminohexane, 1,8-diaminooctane, 1,11-diaminoundecane, 1,12-diaminododecane, 4-aminomethyl-1,8-octanediamine, diethylenetriamine, triethylene-tetramine, 1-amino, 3,3,5-trimethyl-5-aminomethyl-cyclohexane (IPDA), 1,4-cyclohexanediamine, 2,4- and/or 2,6-hexahydrotoluenediamine (H6TDA), isopropyl-2,4-diaminocyclohexane and/or isopropyl-2,6-diaminocyclohexane, 2,4'- and/or 4,4'diaminodicyclohexylmethane (PACM), 3,3'dimethyl-4,4'diaminodicyclohexylmethane, or amine terminated polyether polyols (e.g. Jeffamine® D, ED, EDR or T series commercially available from Huntsman).

In a preferred embodiment of the invention the primary di- or polyamine is a primary diamine, preferably a (cyclo)aliphatic diamine. More preferably, the primary di- or polyamine is 1,5-diaminopentane, 1,6-diaminohexane, 2,4- and/or 2,6-hexahydrotoluenediamine, 2,4'- and/or 4,4'diaminodicyclohexylmethane, 3,3,5-trimethyl-5-aminomethyl-cyclohexane or mixtures thereof. The advantage of using such aliphatic diamines is that they are less likely than polyamines or polyetheramines to deactivate the catalyst used in the reaction with CO₂ and H₂ and that they cannot undergo ring hydrogenation, causing the formation of unwanted side products.

Moreover, this embodiment has the beneficial effect that discoloration of the further prepared polyurethane or polyurea polymers can be significantly reduced.

In a preferred embodiment of the invention, the catalyst used in the carbonylation reaction is a heterogeneous catalyst containing one or more transition metals. Preferably, the metals are present in their metallic form or in form of their oxides and they are optionally supported on carbon or on metal oxides such as alumina, silica, titanium dioxide, zirconium dioxide or mixtures of such metal oxides. In a more preferred embodiment of the invention, the active metal component of the catalyst is a metal or a combination of metals selected from the groups 8 - 11 of the periodic table of elements, particularly preferred are Ru, Ag, Au, Ir, Co, Cu and/or Fe.

Another alternatively preferred embodiment of the present invention provides a process for preparing di- or polyformamides using homogeneous catalysts. It is further preferred in this regard that the homogeneous catalyst is selected from the group consisting of one or more transition metal complexes comprising ligands bearing phosphorous, nitrogen, carbon and/or sulfur as donor atoms or mixtures thereof. More preferably, at least one ligand is a pincer type ligand of the type PCP, PNP, NCN or SCS.

According to the present application a "pincer type ligand" stands for a chelating ligand that coordinates via three adjacent atoms to the respective transition metal, for example a "PCP" pincer type ligang coordinates via a phosphorus atom, a carbon atom and another phosphorus atom to the transition metal. Thus, in above mentioned types "PCP, PNP, NCN or SCS" describe the adjacent coordinating atoms and "P" stands for phosphorus atom, "C" stands for carbon atom, "N" stands for nitrogen atom and "S" stands for a sulphur atom.

In a further preferred embodiment the homogenous catalyst is selected from the group consisting of [IrCl(CO)(PPh₃)₂], [RuCl₂(PMe₃)₄], Fe(BF₄)₂*6 H₂O / P(CH₂CH₂PPh₂)₂, [RuCl₂{PMe₂(CH₂)₂]Si(OEt)₃}₃, [RuCl(H)CO(MeN(CH₂CH₂PPh₂)₂)], [RuCl₂(tris[(₂-diphenylphosphino)phenyl]phosphine)(NHC)], wherein NHC is an n-heterocyclic carbene, and [RuCl₂(1,2-bis(diphenylphosphino)ethan)₂] or mixtures thereof, preferably selected from the group consisting of Fe(BF₄)₂*6 H2O / P(CH₂CH₂PPh₂)₂, [RuCl(H)CO(MeN(CH₂H₂PPh₂)₂)], [RuCl₂(tris[(2-diphenylphosphino)phenyl]phosphine)(NHC)], [RuCl₂(1,2-Bis(diphenylphosphino)ethan)₂] and [RuCl₂(PMe₃)_{4]} or mixtures thereof, more preferably selected from the group consisting of [RuCl(H)CO(MeN(CH₂CH₂PPh₂)₂)], [RuCl₂(PMe₃)₄] or [RuCl₂(1,2-Bis(diphenylphosphino)ethan)₂].

While the described homogeneous catalysts exhibit very good catalytic activity, they can be difficult to separate from the reaction medium and therefore may be less economical. Furthermore, they can interfere with the further use of the formamide products. Thus, it is preferred to use so called supported homogeneous catalysts derived from the above described homogeneous catalysts by means of heterogenization. This can be achieved by anchoring the catalyst to a polymer resin, encapsulating it in a porous material or immobilizing them in a non-miscible solvent.

The formation of diformamides according to the present invention is generally performed at a temperature ranging from 20 °C to 350 °C, preferably 50 °C to 200 °C and more preferably between 70 °C and 170 °C. Higher temperatures may result in decomposition of either the catalyst or the amine, whereas at lower temperatures reaction rates become too low for an industrial process.

The pressure may vary widely and range from 1 to 300 bar(a), preferably from 5 to 200 bar(a) and more preferably from 50 to 150 bar(a).

The reaction can be carried out in any reactor known from the state of the art that is suitable for hydrogen service at the given process temperature and process pressure. Suitable reactors are described for example in "Reactor Types and Their Industrial Applications" (Ullmann's Encyclopedia of Industrial Chemistry; DOI: 10.1002/14356007.b04_087). Particularly preferred are stirred vessel reactors or tubular reactors.

The carbon dioxide used as reactant can be used in solid, liquid or gaseous form. It can also be derived from flue gas, optionally after a purification step. The molar ratio of carbon dioxide to amine groups (n(CO₂):n(NH₂)) in the reaction is preferably between 0.01 to 50, more preferably between 0.2 to 10 and very preferably between 1 and 4.

The ratio of partial pressures for hydrogen and carbon dioxide (p(H₂):p(CO₂)) in the reactor can vary, but is generally in the range from 1:1 to 10:1, preferably from 1:1 to 5:1 and more preferably from 1:1 to 2:1.

The reaction usually takes place at a temperature between 0°C and 350 °C and at a pressure between 1 and 300 bar(a). Preferably, the reaction takes place at a temperature between 50 and 200 °C and at a pressure between 5 and 200 bar(a). More preferably, the reaction takes place at a temperature between 70 and 170 °C and at a pressure between 50 and 150 bar(a).

The reaction can be carried out as a batch process or as a continuous process. In case of a batch process, the di- or polyamine and catalyst are charged to the reactor which is then heated to reaction temperature and pressurized by the addition of carbon dioxide and hydrogen.

In a continuous process, all materials are dosed continuously to the reactor and the products are discharged at an outlet port. Only if a heterogeneous catalyst is used, this may be placed in the reactor as a fixed bed and the remaining feed streams are fed continuously to the reactor. However, it is also possible, to create a slurry containing the di- or polyamine and the catalyst and feed this to the reactor.

Whether the use of a solvent is beneficial or not, depends on the di- or polyamine used in the reaction. If the solvent can be avoided then no additional effort is required to obtain the pure product. Thus, if possible, it is preferred not to use a solvent.

However, in some cases the use of a solvent can be advantageous in order to increase the yield. Thus, in another preferred embodiment the reaction is carried out in the presence of a solvent, preferably in the presence of a polar aprotic solvent, more preferably in the presence of dimethyl sulfoxide and/or tetrahydrofurane.

For further processing, the reaction products are usually discharged from the reactor and depressurized. In a batch process it is also possible to depressurize the reactor first before discharging the product from the reactor. The crude products can be purified by known methods for the skilled person in the art or combination of these methods, for example by phase separation, filtration, extraction, crystallization and/or distillation.

A further subject of the present invention relates to the di- or polyformamide, obtained or obtainable by the inventive process.

The synthesized di- and polyformamides can be used as fillers, binders, chain extenders or catalytic agents polyurethane systems or they can be used as raw material for the production of corresponding di- and polyisocyanates as it is described for example in US 6,781,010 B1, US 4,537,726 A or WO2011067369A1.

Thus, another subject of the present invention relates to the use of the inventive di- or polyformamide as a binder in a polyurethane or polyurea formulation.

Another subject of the present invention relates to the use of the inventive di- or polyformamide for the production of NCO terminated prepolymers.

Another subject of the present invention relates to the use of the inventive di- or polyformamide as starting material in a phosgene free process for the production of isocyanates.

Since the inventive di- or polyformamides are very useful reaction partners for isocyanate groups another subject of the present invention is a two-component-system, containing a binder component A), comprising at least one inventive di- or polyformamide, and a crosslinker B), comprising at least one polyisocyanate.

The invention will be illustrated below with the aid of examples and comparative examples, but without being restricted thereto.

### Examples:

The following examples can be carried out in an autoclave. This autoclave is made from stainless steel and suitable to withstand pressures up to 200 bar and has a heating mantle to allow isothermal reaction conditions up to 200 °C. The autoclave is equipped with a pressure meter and a means for stirring the reaction mixture. The top lid of the autoclave contains 2 feed lines for the gaseous reactants.

### Example 1:

Hexamethylene diamine is added to an autoclave together with a heterogeneous catalyst (1% Au on TiO₂ support). The autoclave is closed and heated to 125 °C before pressurization to 140 bar by addition of CO₂ and H₂ (p(CO₂):p(H₂) = 2:1). The autoclave is stirred for 12 h and hexamethylene diformamide is obtained in good yields.

### Example 2:

Hexamethylene diamine is added to an autoclave together with the homogeneous catalyst RuCl₂(dppe)₂ where the ligand dppe refers to 1,2-bis(diphenylphosphino)ethane. The autoclave is closed and heated to 100 °C before pressurization to 160 bar by addition of CO₂ and H₂ (p(CO₂):p(H₂) = 2.5:1). The autoclave is stirred for 10 h and hexamethylene diformamide is obtained in good yields.

### Example 3:

Isophorone diamine is added to an autoclave together with a homogeneous catalyst [RuCl(H)CO(MeN(CH₂CH₂PPh₂)₂)]. The autoclave is closed and heated to 120 °C before pressurization to 150 bar by addition of CO₂ and H₂ (p(CO₂):p(H₂) = 1:1). The autoclave is stirred for 20 h and isophorone diformamide is obtained in good yields.

### Example 4:

Pentamethylene diamine is added to an autoclave together with the homogeneous catalyst RuCl₂(PMe₃)₄. The autoclave is closed and heated to 110 °C before pressurization to 100 bar by addition of CO₂ and H₂ (p(CO₂):p(H₂) = 1.5:1). The autoclave is stirred for 17 h and pentamethylene diformamide is obtained in good yields.

### Example 5:

Hexamethylene diamine is added to an autoclave together with a heterogeneous catalyst (1% Au on TiO₂ support) and THF. The autoclave is closed and heated to 125 °C before pressurization to 140 bar by addition of CO₂ and H₂ (p(CO₂):p(H₂) = 2:1). The autoclave is stirred for 12 h and hexamethylene diformamide is obtained in good yields.

### Example 6:

Isophorone diamine is added to an autoclave together with a homogeneous catalyst [RuCl(H)CO(MeN(CH₂CH₂PPh₂)₂)] and DMSO. The autoclave is closed and heated to 120 °C before pressurization to 150 bar by addition of CO₂ and H₂ (p(CO₂):p(H₂) = 1:1). The autoclave is stirred for 20 h and isophorone diformamide is obtained in good yields.

## Claims

1. Process for preparing at least one di- or polyformamide comprising at least two -NHCHO groups by reacting at least one primary di- or polyamine with carbon dioxide in the presence of hydrogen and at least one catalyst selected from the group consisting of heterogeneous and homogeneous catalysts or mixtures thereof.

2. Process according to claim 1, wherein the primary di- or polyamine is a primary diamine, preferably a (cyclo)aliphatic diamine.

3. Process according to claim 1 or 2, wherein the primary di- or polyamine is 1,5-diaminopentane, 1,6-diaminohexane, 2,4- and/or 2,6-hexahydrotoluenediamine, 2,4'- and/or 4,4'diaminodicyclohexylmethane, 3,3,5-trimethyl-5-aminomethyl-cyclohexane, or mixtures thereof.

4. Process according to any of the claims 1 to 3, wherein the catalyst is a heterogeneous catalyst containing one or more transition metals.

5. Process according to any of the claims 1 to 3, wherein the catalyst is a homogeneous catalyst containing one or more transition metals.

6. Process according to claim 5, wherein the homogeneous catalyst is selected from the group consisting of one or more transition metal complexes comprising ligands bearing phosphorous, nitrogen, carbon and/or sulfur as donor atoms or mixtures thereof.

7. Process according to claim 6, wherein at least one ligand is a pincer type ligand of the type PCP, PNP, NCN or SCS.

8. Process according to claim 5, wherein the homogenous catalyst is selected from the group consisting of [IrCl(CO)(PPh₃)₂], [RuCl₂(PMe₃)₄], Fe(BF₄)₂*6 H₂O / P(CH₂CH₂PPh₂)₂, [RuCl₂{PMe₂(CH₂)₂]Si(OEt)₃}₃, [RuCl(H)CO(MeN(CH₂CH₂PPh₂)₂)], [RuCl₂(tris[(₂-diphenylphosphino)phenyl]phosphine)(NHC)], wherein NHC is an n-heterocyclic carbene, and [RuCl₂(1,2-bis(diphenylphosphino)ethan)₂] or mixtures thereof, preferably selected from the group consisting of Fe(BF₄)₂*6 H2O / P(CH₂CH₂PPh₂)₂, [RuCl(H)CO(MeN(CH₂CH₂PPh₂)₂)], [RuCl₂(tris[(2-diphenylphosphino)phenyl]phosphine)(NHC)], [RuCl₂(1,2-Bis(diphenylphosphino)ethan)₂] and [RuCl₂(PMe₃)_{4]} or mixtures thereof, more preferably selected from the group consisting of [RuCl(H)CO(MeN(CH₂CH₂PPh₂)₂)], [RuCl₂(PMe₃)_{4]} or [RuCl₂(1,2-Bis(diphenylphosphino)ethan)₂].

9. Process according to any of the preceding claims, wherein the reaction is carried out in the presence of a solvent, preferably in the presence of a polar aprotic solvent, more preferably in the presence of dimethyl sulfoxide and/or tetrahydrofurane.

10. Di- or polyformamide, obtained or obtainable by a process according to any of the preceding claims.

11. Use of a di- or polyformamide obtained according to the process of any of the claims 1 to 9 as a binder in a polyurethane or polyurea formulation.

12. Use of a di- or polyformamide obtained according to the process of any of the claims 1 to 9 for the production of NCO terminated prepolymers.

13. Use of a di- or polyformamide obtained according to the process of any of the claims 1 to 9 as starting material in a phosgene free process for the production of isocyanates.

14. Two-component-system, containing a binder component A), comprising at least one di- or polyformamide according to claim 10, and a crosslinker component B), comprising at least one polyisocyanate.
